# EUROPEAN PATENT APPLICATION

(11) **EP 2 871 183 A1**
(43) Date of publication of application: **13.05.2015**
(21) Application number: 13814001.7
(22) Date of filing: 02.07.2013
(51) Int. Cl.: C07D 309/14, C07D 413/12, A61K 31/351, A61P 25/00, G01N 21/64, G01N 24/08

(54) **USE OF SULPHATED GLYCOLIPIDS AS PROMOTERS OF NEURITIC GROWTH AND MYELINATION AND INHIBITORS OF THE PROLIFERATION OF ASTROGLIA AND MICROGLIA**

(30) Priority: 04.07.2012 ES 201231045
(71) Applicant: Consejo Superior De Investigaciones Científicas (CSIC), 28006 Madrid (ES); Fundación del Hospital Nacional de Parapléjicos Para la Investigación y la Integración, 45071 Toledo (ES)
(72) Inventor: NIETO SANPEDRO, Manuel, E-28002 Madrid (ES); FERNÁNDEZ MAYORALAS, Alfonso, E-28006 Madrid (ES); DONCEL PÉREZ, Ernesto, E-45071 Toledo (ES); GARCÍA ÁLVAREZ, Isabel, E-45071 Toledo (ES)
(74) Representative: Durán-Corretjer, S.L.P.
(86) International application number: PCT/ES2013/070456
(87) International publication number: WO 2014/006250

(57) **Abstract**

The invention relates to the use of a compound having formula I, in which R¹ is an alkenyl group C₅-C₂₅ containing one or more double carbon-carbon bonds, R² is selected independently from the group consisting of methyl and fluorinated methyl, and R³ and/or R⁴ is a SO₃M group, in which M is selected from the group consisting of hydrogen, alkali metal, ammonium and quaternary amine, the other being hydrogen or acyl, for the production of a drug for the treatment of central nervous system disorders selected from the group consisting of lesions caused by CNS trauma, demyelinating diseases, neuro-inflammatory diseases and mental disorders caused a low level of BDNF. The invention also relates to compounds having formula I, to methods for producing said compounds and to compositions containing same.

## Description

### TECHNICAL SECTOR

The present invention relates to the use of sulphated glycolipids having formula (I) for the treatment of diseases of the central nervous system. These compounds act as astrocyte and microglia inhibitors. They also promote neuritic outward growth, dendrites and axons, oligodendroglial volume and myelin synthesis; as well as detection of their interaction with RhoGDIα, a regulator protein of the RhoGTPase family.

### PRIOR ART

Lesions due to trauma to the central nervous system (CNS) are of increasing economic and social importance worldwide. The incidence of medullar damage alone is between 25 and 30 new cases per million residents per year in Spain, which means over a thousand new cases each year. Acute patients are observed to be young people, a fact reflected in the age of those admitted to hospital where 80% are between 15 and 39 years old. An annual report produced in 2007 by the Medical Department of the National Paraplegic Hospital (Hospital Nacional de Parapléjicos) states that high lesions occur in these individuals, that is, a greater number of tetraplegics compared to paraplegics. Specifically, 48% were reported to be cervical lesions compared to 45% for dorsal lesions and 7% for lumbar lesions, although the number of tetraplegics compared with paraplegics has fallen over the past five years. In addition, the percentage of men is far greater than women, specifically 80% men and 20% women.

The Toledo National Paraplegic Hospital (HNPT) has estimated the average cost of hospitalising each patient with medullary damage at €60,000, with an average period of recovery of nine months from admission to medical discharge. The presentation of 1000 new cases of paraplegia or tetraplegia implies an annual cost of €60,000,000, in addition to that for chronic patients receiving treatment, who have a similar life expectancy to the general population. These data from the HNPT are comparable to current figures in developed countries. Even though the number of deaths due to road accidents is clearly falling, this does not mean that the number of patients with medullary injury is decreasing. These accidents are the main cause of cervical lesions leading to total paralysis and the trend of these pathologies is rising.

Damage due to laceration or damage that produces spinal cord cavitation are typical of these traumatic lesions and affect the axons of the central nervous system (CNS). Even a small loss of grey matter can lead to devastating consequences for the patient (M.B. Bunge, Neuroscientist 7, 325 (Aug. 2001; J.H. Kaas et al., Exp Neurol 209, 407 (Feb. 2008)). As well as the initial neuronal death produced by the trauma, anatomical and functional recovery of the damaged area does not occur spontaneously because the growth cones of the damaged axons collapse, withdraw and degenerate. An inhibitory tissue, known as a glial scar, forms in the damaged area which inhibits axon growth and does not allow the passage of regenerative fibres through the lesion. Components of myelin, the extracellular matrix, and the presence of reactive glia, that is, astrocytes and microglia, are responsible for the inhibitory nature of the glial scar (S.K. Kostyk, et al., Neuroscience 156, 498 (Oct 15, 2008); H. Beck, M. Semisch, C. Culmsee, N. Plesnila, A.K. Hatzopoulos, Am J Pathol 173, 77 (Jul. 2008)).

Axonal regeneration in the damaged CNS is largely precluded due to specific inhibitors which accumulate in the lesion site and severely limit functional recovery (G. Yiu, Z. He, Nat Rev Neurosci 7, 617 (Aug. 2006); S. Watkins, M. Yunge, D. Jones, E. Kiely, A.J. Petros, J Pediatr Surg 36, 654, (Apr. 2001)). Inhibitory proteins such as NogoA, MOgp (myelin oligodendrocyte glycoprotein), and MAG (myelin-associated glycoprotein) are expressed by the oligodendroglia. These may be associated with the Nogo receptor (NgR), which in turn may form a complex with the p75 receptor on the surface of neurons. This interaction leads to signal transduction at the cytosol of the neuron, where the RhoA GTPase is activated, and eventually blocks axonal growth (A.A. Vyas, O. Blixt, J.C. Paulson, R.L. Schnaar, J Biol Chem 280, 16305 (Apr 22, 2005); A.A. Vyas et al., Proc Natl Acad Sci U S A 99, 8412 (Jun 11, 2002); T. Yamashita, Brain Nerve 59, 1347 (Dec. 2007)). The search for molecules that inhibit RhoA activity is therefore a promising field for the application of therapies that lead to the repair of the damaged CNS (R.E. Gross, Q. Mei, C.A. Gutekunst, E. Torre, Cell Transplant 16, 245 (2007); S. Mi Cytokine Growth Factor Rev 19, 245 (Jun-Aug 2008)).

In view of the above context, modulation of the Rho family of GTPases, to which CDC42 and Rac also belong, as well as RhoA, is of great interest (E. Boulter et al., Nat Cell Biol 12, 477 (May); M. Gorovoy et al., Circ Res 101, 50 (Jul 6, 2007)).

In an earlier investigation, the applicants synthesised compounds derived from glucosamine with lipophilic groups at the anomeric position and sulphate groups at other positions of the sugar. Using these synthetic molecules, an effective inhibition of the proliferation, growth or migration of astroblasts in culture was observed, processes in which these GTPases are involved. The inhibition by these glycosides of the expression of genes that directly modulate the activity of Rho GTPases was also observed (M. Nieto-Samppedro, E. Doncel, A. Fernández, Expert Opin Ther Pat 14, 487 (2004)).

In a further investigation, the applicants disclose that other derivatives of glucosamine with lipophilic groups at the anomeric position and sulphate groups, sulphated glycolipids having formula I, are capable of inhibiting melanoma and glioma growth (I. Garcia-Alvarez et al., J Med Chem 50, 364 (Jan 25, 2007)). These processes are irreversible as the transformed cells enter apoptosis, the DNA being fragmented (I. Garcia-Alvarez et al., J Med Chem 52, 1263-1267 (2009)). However, in the tests described in this patent application it was observed that the cells affected by the treatment, both astrocytes and microglia, recovered when said treatment was withdrawn, meaning that the inhibitory mechanism is different from that of the transformed cells.

In oncological processes cell proliferation is promoted and hence the production of soluble factors, for example EGF, for the signalling of the transformed cells. However, in normal neural cells following a lesion, growth factor levels decrease drastically and a large number of inhibitory signals are produced (J. Dill et al., J Neurosci 28 (36): 8914-28 (2008)). One of the objects of the present invention is to provide a compound that inhibits this inhibition, which fundamentally comes from the glial cells, thus causing activation of physiological recovery in the affected area of the CNS. In this way, endogenous levels of soluble factors such as brain-derived neurotrophic factor (BDNF) are increased as they are not consumed by the glial cells, allowing that these factors reach the other cell types involved in the transmission and preservation of nerve impulses, in other words, neurons and oligodendrocytes.

### DESCRIPTION OF THE INVENTION

In this invention the use of synthetic glycolipids which promote axon growth, increase myelin production in oligodendrocytes and inhibit astroglial and microglial cell division is disclosed. It is also demonstrated that this behaviour is due to a direct interaction with the RhoGDIα protein, which forms part of the signalling cascade for RhoGTPases. These compounds are stable, soluble in polar solvents and their effect disappears on being eliminated.

In a first aspect, the present invention relates to the use of a compound having formula I 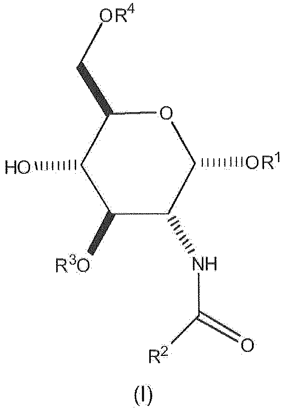 in which
R¹ is an alkenyl residue C₅-C₂₅ containing one or more double carbon-carbon bonds,
R² is selected from the group consisting of methyl and fluorinated methyl, and
at least one of the substitutes R³ or R⁴ is an SO₃M group, in which M is selected from the group consisting of hydrogen, alkali metal, ammonium and quaternary amine, and the other substitute, if it is not an SO₃M group, is selected from the group consisting of hydrogen and acyl;
for the production of a drug for the treatment of central nervous system disorders in which the glial cells and RhoGTPases are involved. Preferably, these central nervous system disorders are selected from the group consisting of lesions caused by CNS trauma, demyelinating diseases, neuro-inflammatory diseases and mental disorders caused by low BDNF levels.

In preferred embodiments of the present invention, a demyelinating disease may be Guillain-Barré syndrome or multiple sclerosis, whereas a neuro-inflammatory disease may be Alzheimer's disease or Parkinson's disease. In addition, mental disorders may be suicidal tendencies or drug dependency.

The term 'fluorinated methyl' refers to a fluoromethyl, difluoromethyl or trifluoromethyl group.

In the present invention, the term 'acyl' refers to a linear or branched carboxylic acid radical, which has from 1 to 6 carbon atoms, and which adheres to the rest of the molecule by means of an ester bond such as, as a non-limiting example, acetyl, propanoyl, butanoyl or pentanoyl.

The term 'quaternary amine' refers in the present invention to an NRₐR_{b}R_{c}R_{d} radical, where Rₐ, R_{b}, R_{c} and R_{d} are selected independently from the group consisting of C₁-C₆, alkyl.

In a preferred embodiment, the present invention relates to the use of the compound having formula I as described in this first aspect of the invention to produce a drug for the treatment of lesions caused by central nervous system (CNS) trauma, preferably for the treatment of cervical, dorsal or lumbar lesions.

In a preferred embodiment, R¹ is selected from the group consisting of octadec-9-enyl and geranylgeranyl having formula:

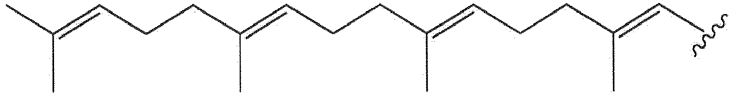

The octadec-9-enyl group is also known as oleyl.

In another preferred embodiment, R³ and R⁴ are both SO₃M, in which M is selected from the group consisting of hydrogen, alkali metal, ammonium and quaternary amine. Preferably, M is an alkali metal selected from the group consisting of sodium and potassium.

In a yet more preferred embodiment, R¹ is selected from the group consisting of octadec-9-enyl and geranylgeranyl as described in the previous paragraphs.

In another preferred embodiment, the present invention relates to the use of the compound having formula I as disclosed in this patent application, in which R¹ is octadec-9-enyl, R² is methyl, R³ is hydrogen and R⁴ is SO₃M, and in which M is an alkali metal selected from the group consisting of sodium and potassium.

In addition, the present invention also relates to a compound having formula I as described in the first aspect for the treatment of central nervous system disorders in which glial cells and RhoGTPases are involved, preferably selected from the group consisting of lesions caused by CNS trauma, demyelinating diseases, neuro-inflammatory diseases and mental disorders caused by low BDNF levels; and in a yet more preferred embodiment, for the treatment of lesions due to central nervous system (CNS) trauma.

Furthermore, the present invention also relates to a method for the treatment of central nervous system disorders where the glial cells and RhoGTPases are involved, preferably selected from the group consisting of lesions caused by CNS trauma, demyelinating diseases, neuro-inflammatory diseases and mental disorders caused by low BDNF levels, where said method comprises administering a therapeutically effective amount of a compound having formula I as described in the first aspect of the invention. In a still more preferred embodiment, the present invention relates to a method for the treatment of lesions caused by central nervous system trauma.

In a second aspect, the present invention relates to a compound having formula I, characterised in that:
R¹ is an alkenyl residue C₅-C₂₅ containing one or more double carbon-carbon bonds,
R² is selected from the group consisting of methyl and fluorinated methyl, and
at least one of the substitutes R³ or R⁴ is an SO₃M group, in which M is selected from the group consisting of hydrogen, alkali metal, ammonium and quaternary amine, and the other substitute, if it is not an SO₃M group, is selected from the group consisting of hydrogen and acyl; provided that R² is methyl, and R³ and R⁴ are SO₃M groups.

In a preferred embodiment, the present invention relates to the compound having formula I, in which R¹ is selected from the group consisting of octadec-9-enyl and geranylgeranyl having formula:

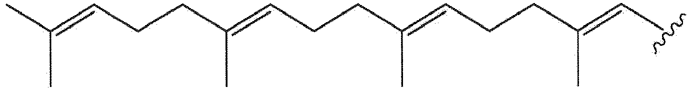

In another preferred embodiment, the present invention relates to the compound having formula I in which R² is a trifluoromethyl group. Preferably, R¹ is also selected from the group consisting of octadec-9-enyl and geranylgeranyl.

In another preferred embodiment, the present invention relates to the compound having formula I in which R³ and R⁴ are both SO₃M groups. Preferably, R¹ is also selected from the group consisting of octadec-9-enyl and geranylgeranyl.

In a third aspect, the present invention relates to a method for producing the compound having formula I as described in the second aspect of the present invention, in which R³ and R⁴ are SO₃M and M is selected from the group consisting of hydrogen, alkali metal, ammonium and quaternary amine, characterised in that it comprises:
a) reaction of a compound having formula II with an alcohol having formula III in the presence of an acid to produce a compound having formula IV. 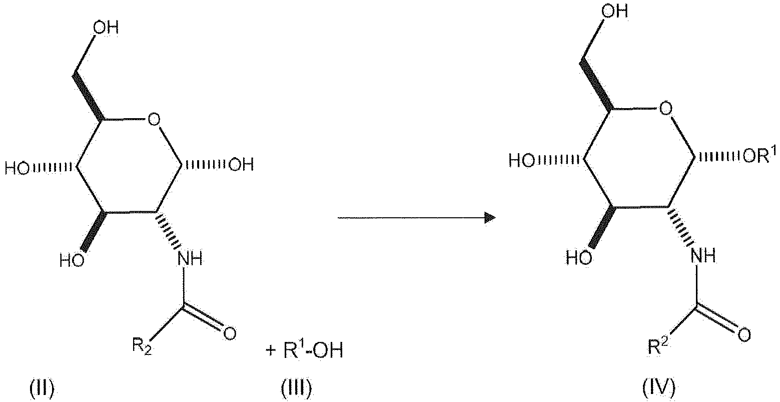 in which
   R¹ is an alkenyl residue C₅-C₂₅ containing one or more double carbon-carbon bonds, and
   R² is selected from the group consisting of methyl and fluorinated methyl, and
b) treating the compound VI with a sulphating agent to produce the compound having formula I in which R³ and R⁴ are SO₃M.

In a preferred embodiment the acid is protic acid. Preferably, the protic acid may be selected from the group consisting of sulphuric acid, p-toluenesulphonic acid, camphorsulphonic acid, trifluoromethanesulphonic acid, hydrochloric acid, hydrobromic acid and hydriodic acid. In a more preferred embodiment, the protic acid may be sulphuric acid adsorbed on silica.

A sulphating agent is understood to be a reagent or mixture of reagents capable of generating an SO₃⁻ group, for example SO₃-triethylamine, SO₃-trimethylamine or SO₃-pyridine. In a preferred embodiment, the sulphating agent is SO₃-pyridine.

The present invention also relates to the method of producing a compound having formula I as described in the second aspect of this patent application, in which R³ is hydrogen, R⁴ is SO₃M and M is selected from the group consisting of hydrogen, alkali metal, ammonium and quaternary amine, characterised in that it comprises:
a') reaction of a compound having formula IV with 2,3-butaneodione and ethanol, in the presence of a protic acid to produce a compound having formula V, 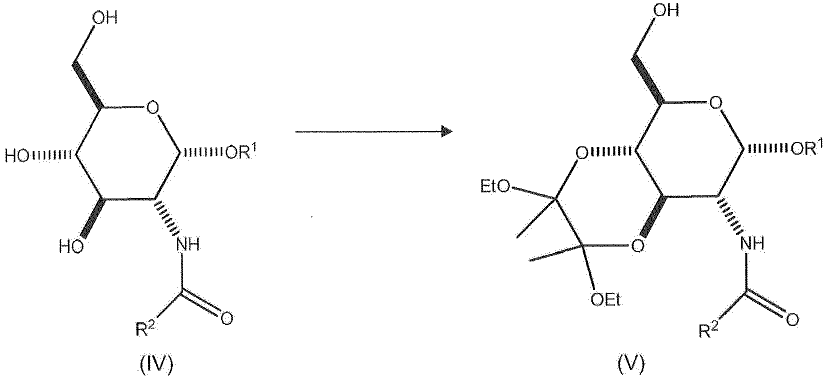 in which
   R¹ is an alkenyl residue C₅-C₂₅ containing one or more double carbon-carbon bonds,
   R² is selected from the group consisting of methyl and fluorinated methyl;
b') treating the compound V with a sulphating agent to produce the compound having formula VI; and 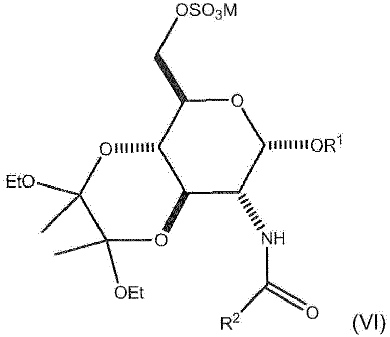
c') hydrolysing the compound VI in the presence of a protic acid to produce the compound having formula I in which R³ is hydrogen and R⁴ is SO₃M, and in which M is selected from the group consisting of hydrogen, alkali metal, ammonium and quaternary amine.

In a preferred embodiment, the compound having formula IV is produced by reaction of a compound having formula II with a compound having formula III as described in step a) of the method for producing a compound having formula I in which R³ and R⁴ are SO₃M.

In another preferred embodiment, the protic acid used in step a') is camphorsulphonic acid.

In another preferred embodiment, the sulphating agent is SO₃-pyridine.

In another preferred embodiment, the protic acid used in step c') is glacial acetic acid.

In a yet more preferred embodiment, the present invention relates to the method for producing a compound having formula I in which R³ is hydrogen and R⁴ is SO₃M and M is selected from the group consisting of hydrogen, alkali metal, ammonium and quaternary amine, in which the protic acid used in step a') is camphorsulphonic acid, the sulphating agent is SO₃-pyridine and the protic acid used in step c') is glacial acetic acid.

The present invention also relates to the method for producing the compound having formula I as described in the second aspect of the present invention, in which R³ is SO₃M, R⁴ is hydrogen and M is selected from the group consisting of hydrogen, alkali metal, ammonium and quaternary amine, characterised in that it comprises:
a") reaction of a compound having formula IV with 2,2-dimethoxypropane in the presence of a protic acid to produce a compound having formula VII; 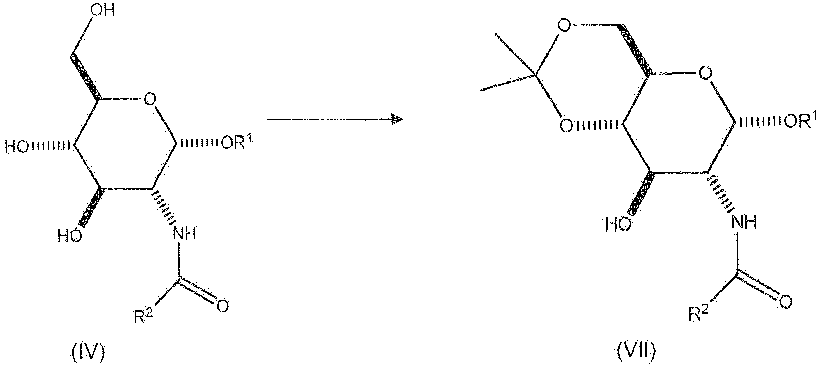 in which
   R¹ is an alkenyl residue C₅-C₂₅ containing one or more double carbon-carbon bonds, and
   R² is selected from the group consisting of methyl and fluorinated methyl;
b") treating the compound VII with a sulphating agent and hydrolysing the acetal group to produce the compound having formula I in which R³ is SO₃M and R⁴ is hydrogen.

In a preferred embodiment, the compound having formula IV is produced by reaction of a compound having formula II with a compound having formula III as described in step a) of the method for producing a compound having formula I in which R³ and R⁴ are SO₃M.

In another preferred embodiment, the protic acid of step a") is p-toluenesulphonic acid.

A sulphating reaction is performed on the compound having formula VII in which the introduction of the sulphate group at position C-3 and hydrolysis of the acetal group take place at the same time, leading to the compound having formula I, in which R³ is SO₃M and R⁴ is hydrogen. In another preferred embodiment, the sulphating agent is SO₃-pyridine.

Any of the three variables in the method for producing the compound having formula I as disclosed in this patent application may comprise the addition of a source of M to produce the compound having formula I in which M is selected from the group consisting of hydrogen, alkali metal, ammonium and quaternary amine. Preferably, the method for producing the compound having formula I comprises the addition of NaOH or KOH, depending on whether M is Na or K respectively, following the sulphating step.

In a fourth aspect, the present invention relates to a pharmaceutical composition which comprises a compound having formula I as described in the second aspect of the present invention.

Some examples of the pharmaceutical compositions of the present invention are solids such as tablets, pills, capsules or solid granules; or liquids such as solutions, syrups, suspensions or emulsions. These pharmaceutical compositions may be suitable for oral, nasal, topical or parenteral administration.

In a preferred embodiment of the present invention, the pharmaceutical compositions are suitable for oral administration, both in solid and liquid form. The possible forms for oral administration may contain conventional excipients known in the pharmaceutical field, such as binding agents (for example, syrup, acacia, gelatine, sorbitol, tragacanth or polyvinyl-pyrrolidone), fillers (for example, lactose, sugar, maize starch, calcium phosphate, sorbitol of glycine), disintegrants (for example, starch, polyvinyl-pyrrolidone or microcrystalline cellulose) or pharmaceutically acceptable surfactants, for example, sodium lauryl sulphate.

Compositions for oral administration may be prepared by conventional methods used in galenic pharmacy, such as mixing and dispersion. Tablets may be covered using methods known in the pharmaceutical industry.

The pharmaceutical compositions may be suitable for parenteral administration, such as sterile solutions, suspensions, or lyophilisates of the compounds of the invention, using the appropriate dose. Suitable excipients may be used, such as pH buffering agents or surfactants.

The above-mentioned formulations may be prepared using conventional methods, such as those described in the pharmacopoeias of the various countries and in other reference texts in the pharmaceutical sector.

The compounds or compositions of the present invention may be administered by any appropriate method, such as intravenous infusion and oral, intraperitoneal or intravenous routes. Oral administration is the preferred method due to its convenience for patients and the chronic nature of the diseases being treated.

The amount of the compound having formula I of the present invention administered will depend on the relative effectiveness of the compound chosen, the severity of the disease being treated and the patient's weight. However, the compounds of this invention will be administered one or more times a day, for example 1, 2, 3 or 4 times daily, with a total dose of between 0.1 and 1000 mg/Kg/day. It is important to bear in mind that it may be necessary to vary the dose, depending on the age and condition of the patient, and also to modify the administration method.

The compounds and compositions of the present invention may be used together with other drugs in combined therapies. The other medicines may form part of the same composition or of a different composition for administration at the same time or at different times.

In a fifth aspect, the present invention relates to a chemically marked derivative having formula VII, in which M is selected from the group consisting of hydrogen, alkali metal, ammonium and quaternary amine. Preferably, M is potassium.

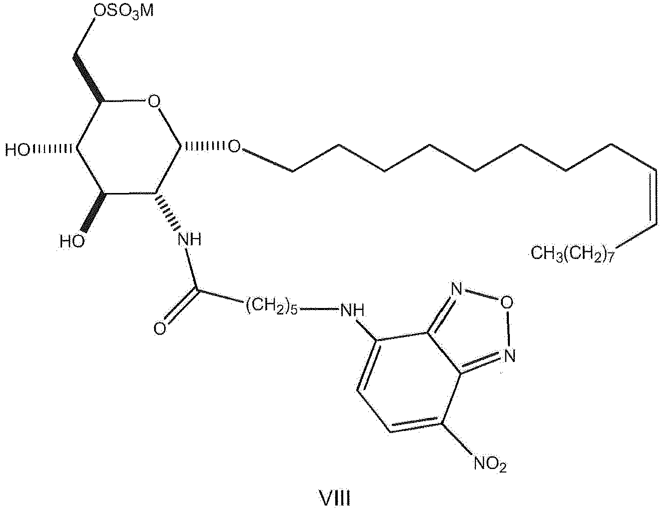

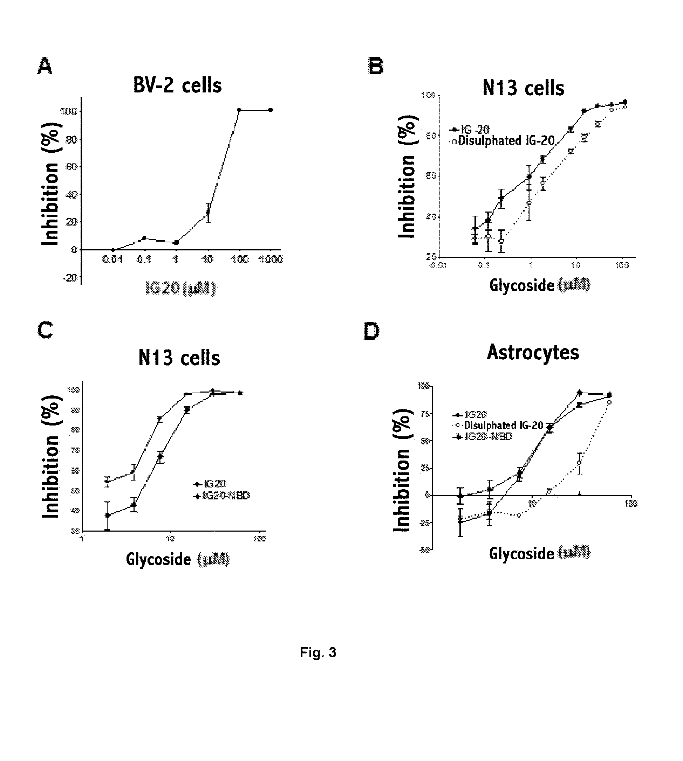

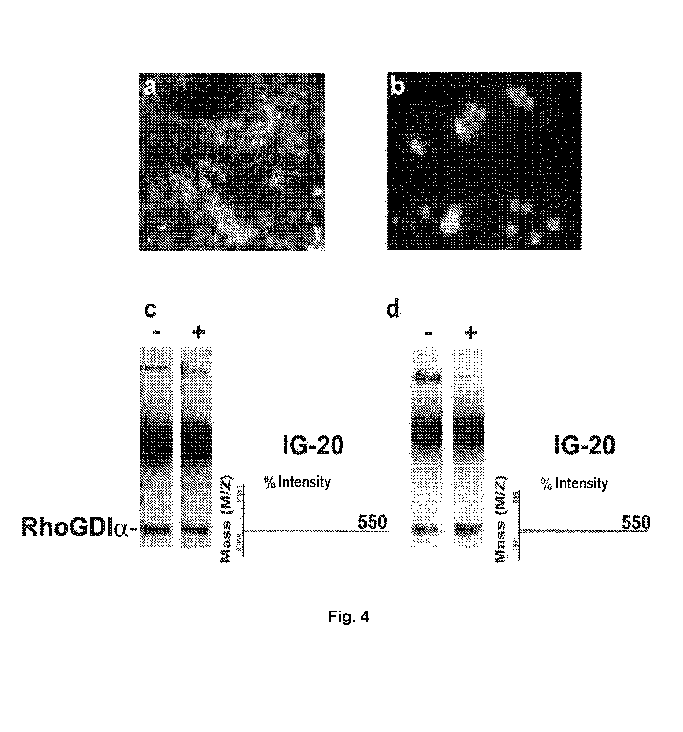

In order to assess the ability of the synthetic sulphated glycolipids to be internalised by the neural cells, a fluorescent compound having formula VIII was prepared. To check whether the inhibitory activity is modified by the presence of the fluorescent group, it was tested as an inhibitor of microglial and astroglial cell proliferation compared with the equivalent without a fluorescent group (see Fig. 3; Table 1 in the Examples), that is, the compound having formula I in which R¹ is an oleyl residue, R² is methyl, R³ is hydrogen and R⁴ is SO₃K. It was subsequently observed that the fluorescent compound was internalised by the neural cells (see Fig. 4 in the Examples).

In a sixth aspect, the present invention also relates to a method for producing the compound having formula VIII as described in this patent application, characterised in that it comprises:
a) hydrolysing the compound having formula I in which R¹ is octadec-9-enyl, R² is trifluoromethyl, R³ is hydrogen and R⁴ is SO₃M to produce a compound having formula IX; 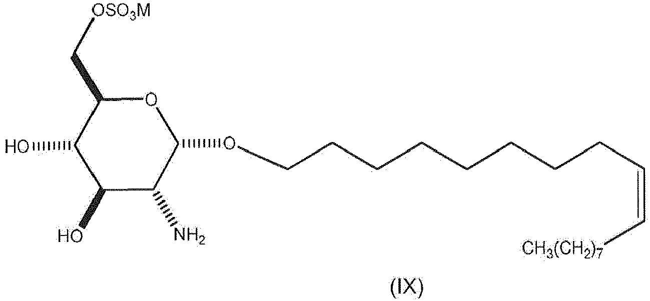
b) acylating the compound having formula IX with a compound having formula X to produce the compound having formula VIII.

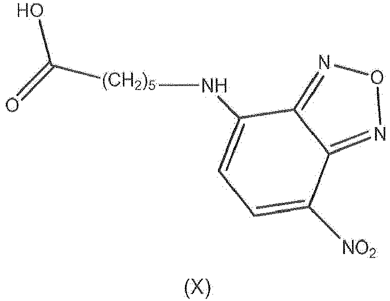

In a preferred embodiment, the compound having formula I that was hydrolysed in step a) is produced using the method described in this patent application.

Preferably, the compound having formula I is hydrolysed with methanol-triethylamine-water (4:1:0.5) to eliminate the trifluoroacetyl group.

The compound having formula X may be produced beforehand from NBD and an acid such as 6-aminohexanoic acid.

In a seventh aspect, the present invention relates to an analytical reagent which comprises the compound having formula I as described in the second aspect, in which R² is fluorinated methyl, or a compound having formula VIII as described in the fifth aspect of the present invention.

If the analytical reagent is used as an imaging agent in *in* vivo analysis techniques, said agent may be suitable for intravenous, intraperitoneal or oral administration.

The analytical reagent that is suitable for parenteral administration may be a solution, a suspension or a lyophilisate of the compound of the present invention, using the appropriate dose. Suitable excipients may be used as pH buffering agents or surfactants.

In an eighth aspect, the present invention relates to the use of the compound having formula I as described in the second aspect, in which R² is fluorinated methyl, or a compound having formula VIII as described in the fifth aspect of the present patent application, to produce an analytical reagent for carrying out biological tests.

In a preferred embodiment, the present invention relates to the use of the compound having formula VIII as described in the fifth aspect, to produce an analytical reagent for carrying out biological tests based on fluorescence detection, preferably biodistribution studies.

Marking a bioactive compound with a fluorescent chemical group is very useful in biodistribution studies based on fluorescence detection, as these compounds allow sensitive and quantitative detection of the compound in tissues, cells and sub-cellular structures using fluorescence microscopy.

Furthermore, the present invention also relates to a compound having formula VIII as described in the fifth aspect, for carrying out biological tests based on fluorescence detection.

In addition, the present invention also relates to a method for carrying out biological tests based on fluorescence detection, in which said method comprises administering a compound having formula VIII as described in the fifth aspect.

In another preferred embodiment, the present invention relates to the use of the compound having formula I as described in the second aspect, in which R² is fluorinated methyl, preferably trifluoromethyl, to produce an analytical reagent for carrying out biological tests based on fluorine detection.

As a non-limiting example, a preferred example of the biological tests in which the compound having formula I can be used, where R² is trifluoromethyl, may be biodistribution and metabolism studies based on the detection of ¹⁹F by nuclear magnetic resonance.

Furthermore, the present invention also relates to a compound having formula I as described in the second aspect, where R² is a fluorinated methyl, for carrying out biological tests based on fluorine detection. R² is preferably trifluoromethyl.

Moreover, the present invention also relates to a method for carrying out biological tests based on fluorine detection, where said method comprises administering a compound having formula I as described in the second aspect of the invention, where R² is a fluorinated methyl. R² is preferably trifluoromethyl.

### BRIEF DESCRIPTION OF THE DRAWINGS

Figure 1. Stimulation of neuritic outgrowth of spinal neurons by the compound IG-20, a glycoside which corresponds to the compound having formula I, in which R¹ is oleyl, R² is methyl, R³ is hydrogen and R⁴ is SO₃K.
Figure 2. The glycoside IG-20 promotes oligodendrocyte growth and the expression of myelin, while inhibiting astroglia.
Figure 3. The compounds IG20, IG20-disulphate and IG20 NBD inhibit microglial and astroglial cell proliferation. The compounds IG20, IG20-disulphate and IG20-NBD correspond to compounds 3, 8 and 11 respectively of the synthesis examples.
Figure 4. The glycoside IG-20 interacts with the RhoGDIα protein in the cytosol of the glial cells.

### EXAMPLES

The invention is illustrated below using tests carried out by the inventors, which demonstrate the specificity and effectiveness of the compounds of the present invention.

### General method for preparing the compounds having formula I

Derivatives having the general formula I were prepared from the monosaccharide N-acetyl-glucosamine or D-glucosamine hydrochloride: 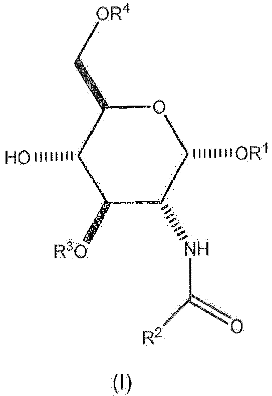 in which
R¹ is an alkenyl residue C₅-C₂₅ containing one or more double carbon-carbon bonds,
R² is selected independently from the group consisting of methyl and fluorinated methyl, and
at least one of the substitutes R³ or R⁴ is an SO₃M group, in which M is selected from the group consisting of hydrogen, alkali metal, ammonium and quaternary amine, and the other substitute, if it is not an SO₃M group, is selected from the group consisting of hydrogen and acyl.

The preparation of the compounds having formula I in which R² is methyl began with the reaction of N-acetyl-glucosamine with an unsaturated hydrocarbon chain alcohol to give the corresponding glycoside. From this glycoside, which had three free hydroxyl groups, two hydroxyls were selectively protected, followed by treatment with a sulphating agent and elimination of the protective group, producing the compound having formula I, where R² is a methyl, and R³ or R⁴ is an SO₃M group.

Alternatively, treatment of the glycoside with a sulphating agent allows the compound having formula I to be produced, in which R² is a methyl, and R³ or R⁴ are both an SO₃M group.

### Preparation of oleyl N-acetyl-α-D-glucosaminide (1)

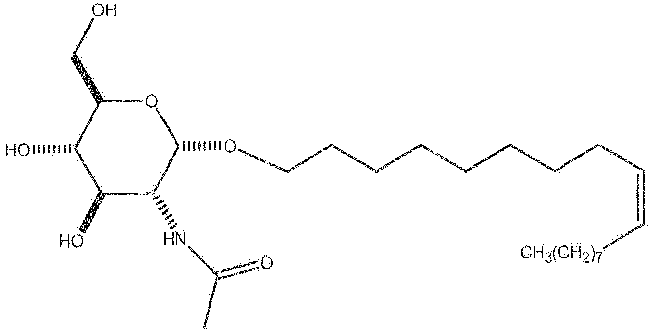

N-acetyl-glucosamine (500 mg, 2.26 mmol) and oleic alcohol (85%) (4.1 mL, 11.3 mmol) were dissolved in nitromethane (2 mL) under argon atmosphere. Next, H₂SO₄ adsorbed on silica (50 mg) was added and the mixture was stirred for 4 hours at 100°C. At the end of this period the reaction mixture was cooled to ambient temperature and the product was isolated using a column of silica gel (AcOEt-MeOH 10:0→10:1), producing a pure substance **1** (405 mg, 38%). [α]_{D}: +103.1° (c 0.95, MeOH). ¹H NMR (300 MHz, CD₃OD: o 5.4-5.3 (m, 2H), 4.74 (d, 1H, *J*=3.5 Hz), 3.9-3.8 (m, 2H), 3.7-3.5 (m, 4H), 3.4-3.3 (m, 2H), 2.0-1.9 (m, 4H), 1.94 (s, 3H), 1.6-1.5 (m, 2H), 1.3-1.2 (m, 22H), 0.86 (t, 3H, *J*=7.2 Hz). ¹³C NMR (75 MHz, CD₃OD) : o

172.42, 129.70, 129.66, 97.29, 72.55, 71.65, 71.22, 67.79, 61.56, 54.39, 32.07, 31.92-28.33, 27.00, 26.10, 23.7, 13.33. MS (ES) m/z (calcd 471.4): 472.4 (M+1), 473.4 (M+2). Anal. Calcd for C₂₆H₄₉NO₆ : C 66.21, H 10.47, N 2.97. Found: C 65.99, H 10.31, N 2.84.

The catalyst acid H₂SO₄ adsorbed on silica (35%) had previously been prepared. To do this, 10 g of silica gel (200-325 mesh) were resuspended in ethyl ether (50 mL) and concentrated H₂SO₄ (3 mL) was slowly added, drop by drop, while stirring. During the adsorption process, which lasted 10 min., the suspension was stirred gently at ambient temperature. At the end of this period, the dissolving agent was eliminated by distillation and the silica was dried at low pressure (2-4 mbar), while being heated to 60°C for 3 hours.

### Preparation of potash from oleyl N-acetyl-3,4-O-(2,3-diethoxy-but-2,3-di-yl)-6-O-(oxo-sulphonyl)-α-D-glucosaminide (2)

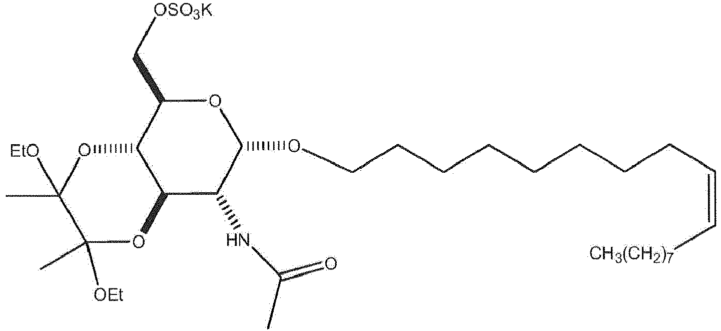

A solution of 1 (100 mg, 0.21 mmol) in ethanol (1.5 mL) was treated with 2,3-butanedione (41 µL, 0.47 mmol), camphorsulphonic acid (10 mg, 0.04 mmol) and triethyl orthoformate (0.23 mL, 1.4 mmol) under argon. The mixture was stirred at 60°C for 3.5 hours. After cooling, the mixture was neutralised with triethylamine, concentrated and the reaction product was purified by chromatography in a column of silica gel (hexane-AcOEt, 1:1 → 0:1), to give a solid (98 mg), which was dissolved in anhydrous pyridine (5 mL). The SO₃-pyridine complex (509 mg, 3.20 mmol) was added, while stirring at ambient temperature, under argon for 1 hour. At the end of this period, the mixture was concentrated and the residue was dissolved in methanol-H₂O (2:1, 7 mL). This residue was neutralised with a solution of KOH 0.5 M, concentrated and purified by chromatography in a column of silica gel (CH₂Cl₂-methanol, 6:1), to give 2 (95 mg, 61%). ¹H NMR (200 MHz, CD₃OD) : δ 5.4-5.3 (m, 2H), 4.83 (d, 1H, *J*=3.4 Hz), 4.6-4.4 (m, 2H), 4.3-3.4 (m, 8H), 2.2-2.0 (m, 7H), 1.6-1.2 (m, 36H), 1.0-0.9 (m, 3H).

### Preparation of potash from oleyl N-acetyl-6-O-(oxo-sulphonyl)-α-D-glucosaminide (3, IG-20)

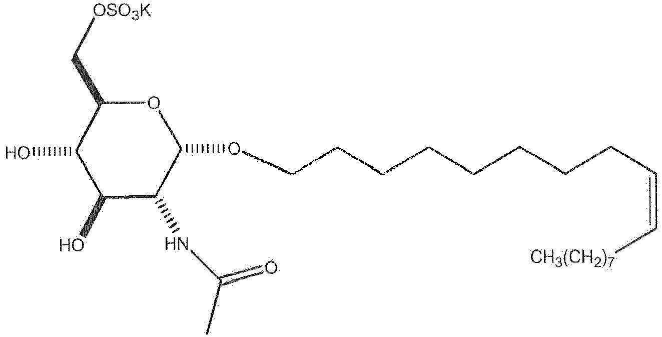

The compound 2 (79 mg, 0.11 mmol) was dissolved in a mixture of acetic acid-H²O (2:1, 10 mL) and stirred for 3 hours at 65°C. The mixture was concentrated and the residue was purified by chromatography in a column of silica gel (CH₂Cl₂-methanol, 5:1 → 4:1) to give **3** (42 mg, 66%), [α]D; +69.0° (c 1.18, MeOH), ¹H NMR (300 MHz, CD₃OD) : δ 5.4-5.3 (m, 2H), 4.76 (d, 1H, *J*=3.4 Hz), 4.26 (dd, 1H, *J*=2.4 Hz, J=11.0 Hz), 4.18 (dd, 1H, *J*=5.6 Hz, *J*=10.7 Hz), 3.89 (dd, 1H, *J*=3.7 Hz, *J*=10.7 Hz), 3.8-3.6 (m, 4H), 3.4-3.3 (m, 2H), 2.0-1.9 (m, 7H), 1.6-1.5 (m, 2H), 1.3-1.2 (m, 22H), 0.9 (t, 3H, *J*=6.8 Hz). MS (ES) m/z (calcd 589.3): 590.3 (M+1). Anal. Calcd for C₂₆H₄₈KNO₉S: C 52.94, H 8.20, N 2.37, S 5.44. Found: C 52.86, H 8.19, N 2.40, S 5.31.

### Preparation of potash from oleyl N-acetyl-3-O-(oxo-sulphonyl)-α-D-glucosaminide (4)

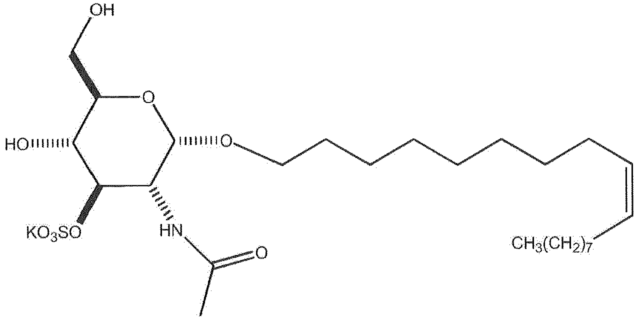

The compound **1** (76 mg, 0.16 mmol), dissolved in anhydrous DMF (1 mL), was treated with 2,2-dimethoxypropane (0.18 mL, 0.8 mmol) and p-toluenesulphonic acid (4 mg). The mixture was stirred for 2 hours at ambient temperature, treated with triethylamine up to neutral pH and concentrated under vacuum. The residue was dissolved in anhydrous pyridine (4.5 mL). The SO₃-pyridine complex (0.5 g, 3.3 mmol) was then added, and it was stirred at ambient temperature under argon for 1.5 hours. At the end of this period, the mixture was concentrated under vacuum, the residue dissolved in methanol-H₂O (2:1, 2 mL), treated with a solution of KOH 0.5 M up to neutral pH and concentrated under vacuum. The residue was extracted with methanol, concentrated under vacuum and purified by chromatography in a column of silica gel (CH₂Cl₂-methanol, 6:1) to give **4** (82.1 mg, 82%). [α]_{D}: +39.4° (c 0.89 MeOH). ¹H NMR (300 MHz, CD₃OD): δ 5.4-5.3 (m, 2H), 4.48 (d, 1H, *J*=3.2 Hz), 4.48 (dd, 1H *J*=10.7 Hz, *J*=11.0 Hz), 3.96 (dd, 1H, *J*=3.7 Hz, *J*=10.7 Hz), 3.8-3.5 (m, 4H), 3.4-3.3 (m, 2H), 2.0-1.9 (m, 7H), 1.6-1.5 (m, 2H), 1.3-1.2 (m, 22H), 0.90 (t, 3H, *J*=7.1 Hz). ¹³C NMR (75 MHz, CD₃OD) : δ 173.69, 130.84, 130.81, 98.32, 80.03, 73.64, 70.85, 69.15, 62.26, 54.10, 33.57, 33.02, 30.9-27.3, 23.70, 22.87, 14.46. MS (ES) m/z (calcd 589.3): 590.2 (M+1). Anal. Calcd for C₂₆H₄₈KNO₉S: C 52.94, H 8.20, N 2.37, S 5.44. Found: C 53.10, H 8.35, N 2.62, S 5.60.

The preparation of the compounds having formula I in which R² is trifluoromethyl began with the reaction of D-glucosamine hydrochloride with *S*-ethyl trifluorothioacetate, to produce *N*-trifluoroacetyl-D-glucosamine with an anomer mixture. The glycosylation reaction of the mixture *N*-trifluoroacetyl α, β-D-glucosamine produced with an unsaturated hydrocarbon chain alcohol, produced the corresponding N-trifluoroacetyl glycoside. Next, it was treated with a sulphating agent to produce the compound having formula I, in which R² is trifluoromethyl, at least one of the substitutes R³ or R⁴ is an SO₃M group, in which M is selected from the group consisting of hydrogen, alkali metal, ammonium and quaternary amine, and the other substitute, if it is not an SO₃M group, is selected from the group consisting of hydrogen and acyl.

### Preparation of N-trifluoroacetyl-α, β-D-glucosamine (5)

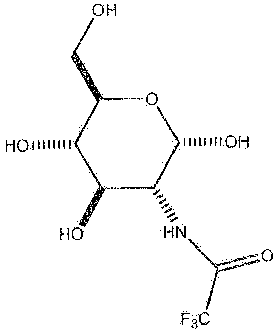

A suspension of D-glucosamine hydrochloride (0.51 g, 2.32 mmol) in anhydrous methanol (2.5 mL) was treated with an equivalent of sodium methoxide in methanol (53 mg of Na dissolved in 0.6 mL of methanol). The mixture was stirred at ambient temperature to produce a transparent solution, with a white precipitate of NaCl at the bottom. *S-*ethyl trifluorothioacetate (SETFA) was added (0.42 mL, 3.26 mmol) and the reaction mixture was stirred at ambient temperature for 17 hours. (TLC: AcOEt-MeOH, 2:1). At the end of this period, the dissolving agent was eliminated at low pressure and the residue extracted with warm acetone. Ethyl ether was added to the acetone extract at 0°C and left to crystallise at 4°C for 12 hours. The crystallised compound was filtered and washed with acetone at 0°C, to give **5** as a white solid. The compound **5** was produced with an anomer mixture, ratio (α/β 7:3) (399 mg, 62%). Mp: 180-184°C. [α]_{D}: +54.6° (c 0.5, MeOH). ¹H NMR (400 MHz, CD₃OD: δ 5.15 (d, <1H, *J*=3.0 Hz), 4.69 (d, <1H, *J*=8.3, Hz), 3.9-3.7 (m, 3H), 3.8-3.6 (m, <2H), 3.53 (dd, <1H, *J*=10.4, 8.4 Hz), 3.04 (dd, 1H, *J*=10.4, 3.6 Hz). ¹³C NMR (100 MHz, CD₃OD): δ 159.2, 119.4, 96.3, 92.0, 78.2, 75.3, 73.2, 72.5, 72.2, 71.9, 62.8, 62.7, 59.1, 56.7. MS (ESI +) m/z (calcd 275.0623): 276.0696 (M+H)⁺, 543.3621 (M+NH₄)⁺. Anal. Calcd for C₈H₁₂F₃NO₆: C 34.92, H 4.40, N 5.09. Found: C 35.00, H 4.15, N 4.52.

### Preparation of oleyl N-trifluoroacetyl-α-D-glucosaminide (6)

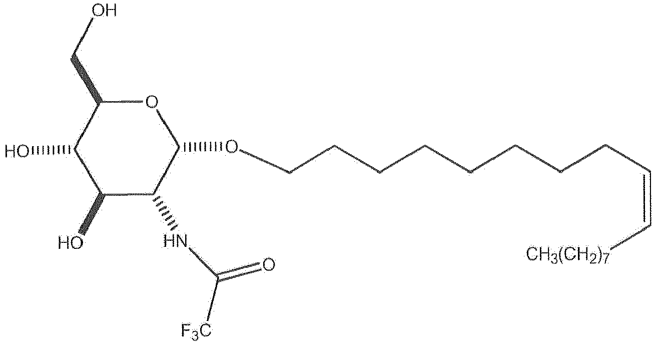

The compound **5** (1.18 g, 4.29 mmol) was dissolved in 85% oleic alcohol (8mL, 21.4 mmol, 5 eq) and treated with H₂SO₄ adsorbed on silica (35%) (236 mg). The reaction mixture was stirred under argon at 180°C for 20 minutes (TLC: AcOEt). At the end of this period, the reaction mixture was cooled to ambient temperature and the residue purified by chromatography in a column of silica gel (Hexane-AcOE2 2:1 → 0:1) to give **6** as a solid (604 mg, 29%). Mp: 118-123°C. [α]_{D}: +86.0° (c 0.5, MeOH). ¹H NMR (400 MHz, CD₃OD: δ 5.4-5.3 (m, 2H), 4.86 (d, 1H, *J*=3.6 Hz), 3.90 (dd, 1H, *J*=10.8, 3.6 Hz), 3.9-3.8 (m, 1H), 3.80 (t, 1H, *J*=1.9 Hz), 3.8-3.7 (m, 2H), 3.60 (ddd, 1H, *J*=9.9, 5.5, 2.3 Hz), 3.5-3.3 (m, 2H), 2.1-1.9 (m, 4H), 1.8-1.5 (m, 2H), 1.5-1.1 (m, 22H), 0.9 (t, 3H, *J*=7.0 Hz). ¹³C NMR (100 MHz, CD₃OD): δ 159.2, (q, *J*=37.5 Hz), 117.5 (q, *J*=287.6 Hz), 130.8, 97.7, 73.8, 72.3, 71.9, 68.98, 62.6, 56.2, 33.1, 30.9, 30.9, 30.6, 30.6, 30.6, 30.5, 30.5, 30.4, 30.3, 28.1, 28.1, 27.3, 23.8, 14.5. MS (ESI +) m/z (calcd 525.3283): 526.3345 (M+H)⁺, 543.3621 (M+NH₄)⁺. Anal. Calcd for C₂₆H₄₆F₃NO₆: C 59.41, H 8.82, N 2.66. Found: C 59.66, H 9.12, N 2.69.

The prior preparation of H₂SO₄ adsorbed on silica takes place in the same way as for producing the compound (1).

### Preparation of potash from oleyl N-trifluoroacetyl-6-O-(oxo-sulphonyl)-α-D-glucosaminide (7)

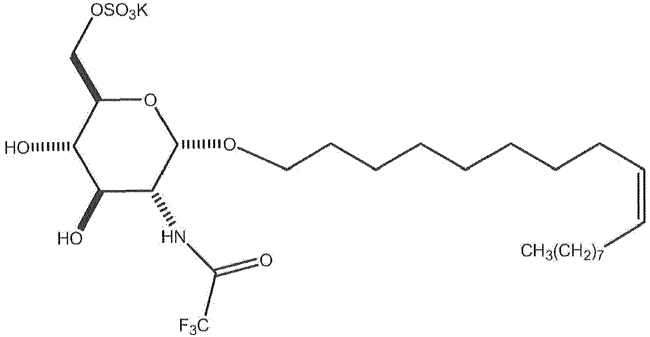

The compound **3** (458 mg, 0.87 mmol) was dissolved in anhydrous pyridine (1mL) and treated with the SO₃-pyridine complex (290 mg, 1.82 mmol). The reaction mixture was stirred under argon at ambient temperature for 1 hour and 20 minutes. At the end of this period, the dissolving agent was eliminated at low pressure and the residue was dissolved in MeOH-H₂O (2:1, 6mL), neutralised with a solution of KOH 0.5 M and concentrated under vacuum. The residue was extracted with methanol, concentrated and purified by chromatography in a column of silica gel (AcOEt/MeOH/Et₃N 1:0:0.005 → 1:0.5:0.005) to give **7,** as a yellow solid (360 mg, 64%). ¹H NMR (300 MHz, CD₃OD: δ 5.42-5.27 (m, 2H), 4.91 (s, 1H), 4.27 (dd, *J*=10.9, 2.2 Hz, 1H), 4.18 (dd, *J*=10.9, 5.1 Hz, 1H), 3.90 (dd, *J*=10.7, 3.5 Hz, 1H), 3.82 (dd, *J*=8.1, 2.8 Hz, 1H), 3.76 (dd, *J*= 4.7, 2.5 Hz, 1H), 3.72 (dd, *J*=8.2, 4.5 Hz, 1H), 3.44 (d, *J*=8.6 Hz, 1H), 3.41-3.34 (m, 2H), 2.03 (m, 4H), 1.57 (m, 2H), 1 .46 - 1. 11 (m, 22H), 0.90 (t, *J*=6.7 Hz, 3H). ¹³C NMR (126 MHz, CD₃OD): δ 157.63, 129.42, 129.39, 96.19, 70.62, 70.45, 70.28, 67.66, 66.67, 54.73, 31.64, 29.49, 29.42, 29.38, 29.37, 29.33, 29.22, 29.20, 29.18, 29.17, 29.15, 29.02, 28.94, 28.90, 26.72, 26.69, 25.83, 22.31, 13.04. MS (ESI -) m/z (calcd 604.2771): 604.2778 (M)⁻.

### Preparation of potash from oleyl N-trifluoroacetyl-3,6-O-di-(oxo-sulphonyl)-α-D-glucosaminide (8)

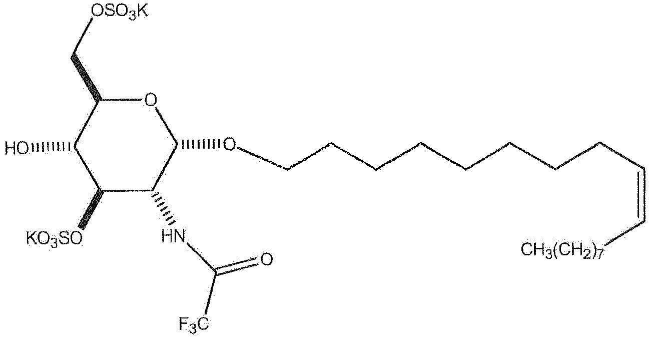

The compound **3** (458 mg, 0.87 mmol) was dissolved in anhydrous pyridine (1 mL) and treated with the SO₃-pyridine complex (290 mg, 1.82 mmol). The reaction mixture was stirred under argon at ambient temperature for 1 hour and 20 minutes. At the end of this period, the dissolving agent was eliminated at low pressure and the residue dissolved in MeOH-H₂O (2:1, 6 mL), neutralised with a solution of KOH 0.5 M and concentrated under vacuum. The residue was extracted with methanol, concentrated and purified by chromatography in a column of silica gel (AcOEt/MeOH/Et₃N 1:0:0.005 → 1:0.5:0.005) to give **8** as a solid (125 mg, 19%). ¹H NMR (300 MHz, CD₃OD: δ 5.42-5.29 (m, 2H), 5.11 (d, *J*=3.5 Hz, 1H), 4.59 (dd, *J*=10.8, 8.9 Hz, 1H), 4.30 (dd, *J*=10.8, 2.0 Hz, 1H), 4.19 (dd, *J*=10.8, 5.4 Hz, 1H), 3.91 (dd, *J*= 5.1, 27 Hz, 1H), 3.89-3.84 (m, 1H), 3.76 (dt, *J*=9.9, 6.1 Hz, 1H), 3.66-3.58 (m, 1H), 3.40 (dt, *J*=10.0, 6.4 Hz, 1H), 2.03 (d, *J*=5.5 Hz, 4H), 1.57 (d, *J*=6.6 Hz, 2H), 1.35-1.25 (m, 22H), 0.90 (t, *J*=6.7 Hz, 3H). ¹³C NMR (75 MHz, CD₃OD) : δ 174.06, 131.37, 131.33, 98.62, 80.09, 72.30, 71.34, 69.66, 68.58, 54.70, 33.53, 31.38, 31.29, 31.19, 31.07, 30.92, 30.86, 30.78, 28.64, 28.58, 27.77, 24.21, 23.30, 14.97. MS (ESI -) m/z (calcd 684.2351): 684.2383 (M)⁻, 341.6207 (M)²⁻.

### Preparation of potash from oleyl 6-O-di-(oxo-sulphonyl)-α-D-glucosaminide (9)

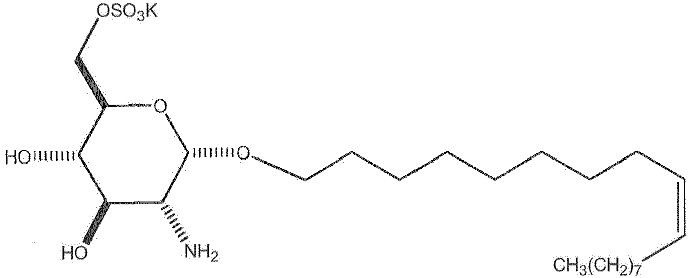

**7** (100 mg, 0.16 mmol) was dissolved in methanol-triethylamine-water 4:1:0.5 (1 mL) and stirred at 50°C for 48 hours. At the end of this period, the mixture was concentrated under vacuum and the residue was purified by chromatography in a column of silica gel (AcOEt-MeOH-Et₃N 1:0:0.005 → 0:1:0.005) to give **9** (60 mg, 71%) as a white solid. R_{f}: 0.23 (AcOEt-MeOH 2:1). [α]_{D}: +36° (c 8.6, MeOH). ¹H NMR (400 MHz, CD₃OD: δ 5.4-5.3 (m, 2H), 4.93 (d, J=3.7 Hz, 1H), 4.22 (dd, J=10.9, 2.1 Hz, 1H), 4.16 (dd, J=11.0, 4.9 Hz, 1H), 3.78-3.74 (m, 1H), 3.73-3.69 (m, 1H), 3.66 (dd, J=10.3, 9.0 Hz, 1H), 3.52-3.42 (m, 1H), 3.42-3.35 (m, 1H), 3.04-2.96 (m, 1H), 2.08-1.92 (m, 4H), 1.62 (m, 2H), 1.42-1.19 (m, 22H), 0.88 (t, 3H). ¹³C NMR (100 MHz, CD₃OD) : δ 130.83, 111.19, 97.52, 72.21, 71.41, 69.41, 67.74, 55.98, 33.07, 30.93, 30.84, 30.80, 30.77, 30.68, 30.65, 30.61, 30.53, 30.45, 30.41, 30.33, 28.17, 23.74, 14.46. HRMS (ESI +) m/z (calcd 508.29 for C₂₄H₄₆NO₇S) : 510.3065 (M-2H)⁺, 532.2763 (M-K-H)⁺.

### Preparation of potash from oleyl 2-[6-(7-nitrobenzofurazan)-amino hexanamide]-6-O-(oxo-sulphonyl)-α-D-glucosaminide (11)

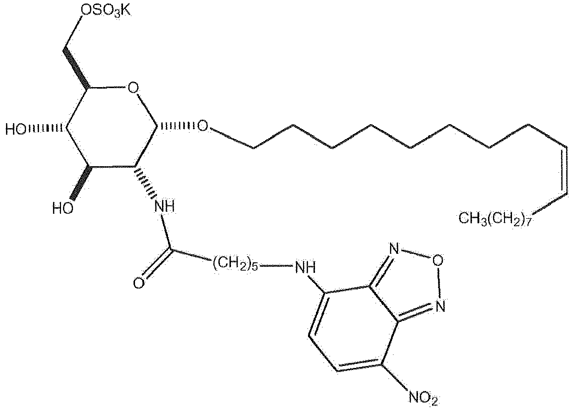

**9** (90.2 mg, 0.16 mmol) was dissolved in anhydrous THF (2 mL) and treated with the acid **10** (72.5 mg, 0.25 mmol), 1-ethyl-3-(3-dimethylaminopropyl)carbodiimide (EDC) (47.2 mg, 0.25 mmol) and 4-dimethylaminopyridine (DMAP) (10.0 mg, 0.08 mmol). The reaction mixture was stirred under argon at ambient temperature for 24 hours. At the end of this period, the dissolving agent was eliminated under vacuum and the residue was purified by chromatography in a column of silica gel (CH₂Cl₂-MeOH-Et₃N, 1:0:0.005 → 0:1:0.005) to give **11** as a white solid (95 mg, 70%). R_{f} = 0.31 (CH₂Cl₂-MeOH 4:1). [α]_{D}: +39.4° (c 11.3, MeOH). ¹H NMR (300 MHz, CD₃OD: δ 8.51 (d, J=8.8 Hz, 1H), 6.35 (d, J=8.9 Hz, 1H), 5.30 (m, 2H), 4.77 (d, J=3.6 Hz, 1H), 4.26 (dd, J=10.7, 2.0 Hz, 1H), 4.17 (dd, J=10.8, 5.3 Hz, 1H), 3.89 (dd, J=10.8, 3.6 Hz, 1H), 3.81 - 3.72 (m, 1H), 3.71-3.68 (m, 1H), 3.65 (d, J=3.0 Hz, 1H), 3.53 (s, 2H), 3.42 (d, J=9.5 Hz, 1H), 3.39-3.33 (m, 1H), 2.29 (t, 2H), 1.98 (s, 4H), 1.87-1.76 (m, 2H), 1.70 (dd, 2H), 1.54 (m, 2H), 1.50 (m, 2H), 1.28 (m, 22H), 0.89 (t, 3H). ¹³C NMR (100 MHz, CD₃OD): δ 175.07, 144.54, 144.48, 137.53, 130.28, 130.20, 129.61, 97.15, 71.28, 70.98, 70.65, 67.81, 67.04, 35.55, 32,43, 31.88, 31.06, 29.94, 28.86, 28.14, 27.49, 26.95, 26,37, 26.28, 26.18, 22.56, 13.30. HRMS (ESI-) m/z (calcd 784.371 for C₃₆H₅₈N₅O₁₂S) : 784.3716 (M)⁻.

### Tests on the therapeutic activity of the compounds of the present invention

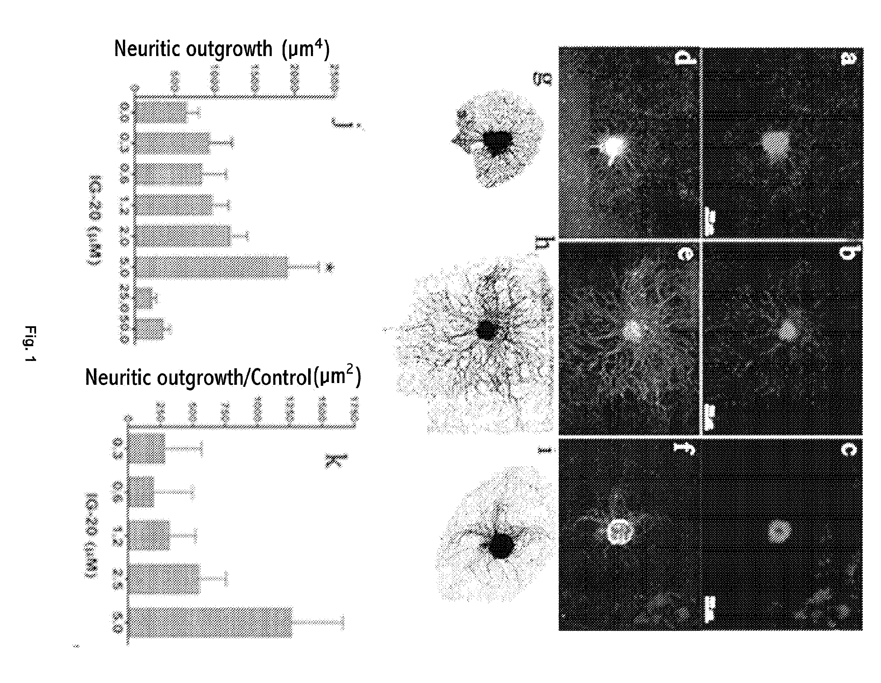

Spinal ganglia (SG) of rat embryos (E15) were dissected and incubated for 12 hours on axon growth-promoting cells (aldynoglia) to allow cellular adhesion of the neurons contained in the SG to the aldynoglia substrate. Next, different concentrations of IG-20, compound **3** produced as described in the synthesis examples, were added. The cocultures of SG/aldynoglia were fixed after 3 days incubation, which revealed the presence of axons, dendrites and somas of the neurons using anti-neurofilament antibody (Fig. 1d, 1e, If). The presence of total cells is shown by the fluorescent signal of the Hoechst-marked nuclei (Fig. 1a, 1b, 1c). The nuclei remote from the central body are aldynoglia cells or those that migrate from the SG. The neurofilament images were extracted (Fig. 1g, 1h, 1i) and measured, and the neuritic growth area was compared to the controls (Fig. 1j, 1k). As a result a profuse and significant increase in neuritic outgrowth was observed at 5 µM of IG-20 (Fig. 1e, 1h) compared with the growth promoted by the control aldynoglia (Fig. 1d, 1g). A five-fold increase [in] this concentration reduced this neuritic outgrowth (Fig. 1f, 1j). A net increase in the neuritic growth area of 1250 µm² more than the control with aldynoglia cells (Fig. 1k) was produced. The average speed of neuritic outgrowth in the presence of aldynoglia, 625 µm/72h corresponding to 8.3 µm/h, was duplicated in the presence of IG-20 (5 µM) up to 1250 µm/72h, which corresponds to 17.36 µm/h. Calibration bars at 500 µm; (*) correspond to P<0.05 (P=0.019).

To study how the compound having formula I affects the growth of oligodendrocytes, the expression of myelin and the inhibition of astroglia, cerebral cortices of postnatal rats P0-P1 were dissected and enzymatically dissociated for cell culture. After two weeks incubation the culture flasks were stirred for 12 hours. The cells in suspension were harvested, the microglia were removed, and then cultured in DMEM plus SBF 10%. It was incubated for 6 hours to facilitate cell adhesion and the IG-20 (30 µM) was added to the treated group. The cells were cultured for two weeks and the medium was replaced every three days. Finally, the cells were fixed for immunocytochemistry and revealed by GFAP, MBP antibody whilst the nuclei were detected by Hoechst.

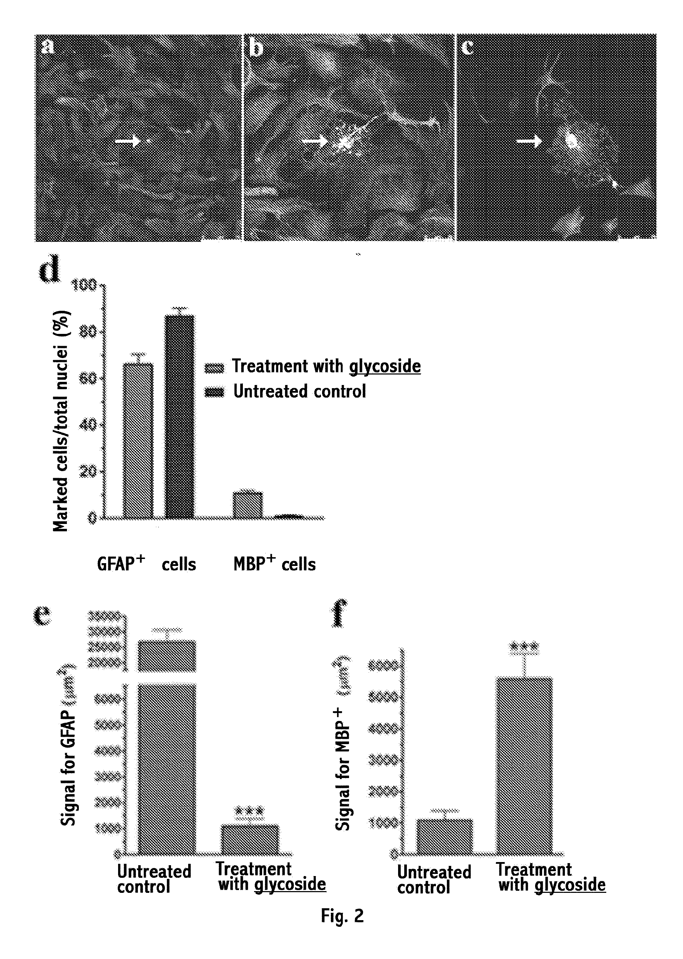

The treatment reduced the proportion of GFAP+ cells, which correspond to astroglia, by 20%, whilst the mature oligodendroglia, MBP+ increased by 10% (Fig. 2d). A dramatic reduction in GFAP marking was observed, compared with the untreated control group, which contrasts with the increase in the MBP signal (Fig. 2e, f). In both cases the differences were very significant. It was notable that the astroglial cells were large compared with the oligodendrocytes, which could scarcely be detected in the untreated controls (arrow in 2a, b). The cultures treated with IG-20 revealed oligodendrocytes that were 3-4 times larger than the controls (arrow in 2c) and profuse arborisation, together with significantly lower GFAP expression. GFAP, *glial fibrillary acidic protein;* MBP, *myelin basic protein;* calibration bars at 75 µm (a, c) and 25 µm (b), as shown; (***) corresponds to P<0.001.

To study the microglial and astroglial cell proliferation two cell lines of murine microglia were used (BV-2, N13), as well as primary astroblasts isolated from cerebral cortices of postnatal rats on day zero (P0). The cells were kept in DMEM plus 10% SBF, grown and treated for testing the inhibition of cell proliferation, as published earlier by the applicants (I. Garcia-Alvarez et al., J Med Chem 50, 364 (Jan 25, 2007)). In both microglial lines effective inhibition of proliferation by IG-20 was observed, with an IC50 of 21.0 and 3.4 µM for BV-2 and N13 cells respectively (Fig. 3A, 3B). Effective inhibition of microglia and astrocytes was also observed (Fig. 3C, 3D) at 7.8 and 11.4 µM for IG-20 combined with the NBD fluorescent group; as was also the case for the derivative of IG-20 with two sulphated groups and in which R² is trifluoromethyl (IG-20 disulphate). It was concluded that the inhibitory activity of IG20-NBD is not affected by the presence of the fluorescent group, as its IC50 is very similar to that obtained for the uncombined glycoside IG-20, which was used as a control in these tests. However, the disulphated IG-20 derivative reduced its activity by three compared with IG-20 in astrocytes, at a concentration of 30 µM compared with 9.3 µM respectively, although microglial inhibition was not affected (Table 1).

**Table 1: Inhibition of microglia and astrocytes by glycosides**

| **Cells** | **Type, species** | **Glycoside** | **IC₅₀ (µM) *** |
|---|---|---|---|
| **BV-2** | Microglia, mouse | IG-20 | 21 ± 3.7 |
| **N13** | Microglia, mouse | IG-20 | 3.4 + 0.1 |
| **N13** | Microglia, mouse | IG20-NBD | 7.8 ± 0.6 |
| **N13** | Microglia, mouse | Disulphated IG-20 | 3.0 ± 0.8 |
| **Astrocytes** | Astroglia, rat | IG-20 | 9.3 ± 0.7 |
| **Astrocytes** | Astroglia, rat | IG20-NBD | 11.4 ± 0.7 |
| **Astrocytes** | Astroglia, rat | Disulphated IG-20 | 30.0 ± 4.8 |

| | | | |
|---|---|---|---|
| (*) **IC₅₀** ± SE | | | |

Primary astrocyte and microglia cells (BV-2) were treated with the glycoside IG-20 at 30 µM, lysed and subjected to cell fractionation by differential centrifugation. The soluble fraction was collected and mixed with lysis buffer (1:1). Anti-RhoGDIα antibody (15 µg) was added and it was incubated with rotation at 4°C overnight. It was immunoprecipitated with Protein *A-Sepharose* (20µl) having been previously washed with PBS and centrifuged for 3 minutes at 1100 rpm. The previous mixture was added to the Protein *A-Sepharose* (1:1). This mixture was incubated with rotation at 4°C for an hour and a half, and afterwards centrifuged for 5 minutes at 2500 rpm at 4°C. After discarding the supernatant, the precipitate was washed three times with lysis buffer. The immunocomplex of the Protein A-*Sepharose* was eluted with five volumes of the elution buffer (0.1 M glycine, pH=2.9) and a neutralising volume of 1M tris-HCl, pH=9. The mixture was centrifuged for five minutes at 2500 rpm at 4°C and the supernatant was collected. The sample immunoprecipitated with anti-RhoGDIα was divided for Western blot analysis and sent to the mass spectrometry department of the Toledo National Paraplegic Hospital.

Fig. 4a and 4b show that the glycoside IG20-NBD penetrates the glial cells and accumulates in the cytosol of astrocytes and microglial cells. No co-localisation signal was observed in the nucleus in either of the two cell types tested. The areas of fluorescence were very weak at the limits of the primary cells (Fig. 4a), with the IG20-NBD signal falling towards the cell membrane. However, in the intracellular areas (Fig. 4a, b) accumulations or dotted areas of IG20-NBD of different size were evident forming aggregates inside the astrocyte and microglia cells.

Furthermore, Fig. 4c and 4d show that the glycoside IG20 interacts directly with the RhoGDIα protein, both in astrocytes (Fig. 4c) and in microglia (Fig. 4d). This was verified by RhoGDIα immunoprecipitation in the microglial cell line BV-2, and also in primary astrocyte cultures. The cultures were incubated with 30 µM of the glycoside IG-20, using untreated cells as a control. Each immunoprecipitated sample was divided for analysis by Western blot (WB) and mass spectrometry (MS). A 27kDa signal was observed in the control (-) and treated (+) cells, corresponding to RhoGDIα, detected with a specific monoclonal antibody for the WB tests. However, only the preparations of cells, astrocytes or BV-2 being treated with IG-20 (+) co-immunoprecipitated with the sulphated glycolipid. This was detected by MS as a peak in the mass to charge ratio of 550 (Fig. 4c, 4d), which corresponds to the synthetic glycoside. The cytoplasmic fractions were enriched in the IG-20 compound, which was weakly detected in the membrane fractions tested (datum not shown). This co-immunoprecipitation of the glycoside IG-20 and RhoGDIα, with the specific anti-RhoGDIα antibody, was evidence of a direct interaction of both molecules in the cytosol.

The results described in Fig. 4 indicate that the sulphated glycolipid IG-20 combines with the RhoGDIα, protein in the cytosol of the glial cells. This sulphated glycolipid could then alter the final component of the BDNF/TrkB-T1/RhoGDIα signalling, which has a very marked effect on microglia and astroglia [20-22]. It was also demonstrated that neuritic outgrowth and myelination (Fig. 1, 2) were promoted by the compound IG-20, which inhibited microglial and astroglial proliferation (Fig. 3 and Table 1), reducing the GFAP signal and the size of the astrocytes (Fig. 2).

## Claims

1. Use of a compound having formula I 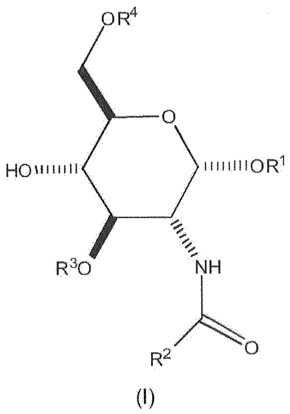 in which
R¹ is an alkenyl residue C₅-C₂₅ containing one or more double carbon-carbon bonds,
R² is selected from the group consisting of methyl and fluorinated methyl, and
at least one of the substitutes R³ or R⁴ is an SO₃M group, in which M is selected from the group consisting of hydrogen, alkali metal, ammonium and quaternary amine, and the other substitute, if it is not an SO₃M group, is selected from the group consisting of hydrogen and acyl;
for the production of a drug for the treatment of central nervous system disorders in which the glial cells and RhoGTPases are involved.

2. Use of a compound having formula I according to claim 1, in which the central nervous system disorders are selected from the group consisting of lesions caused by CNS trauma, demyelinating diseases, neuro-inflammatory diseases and mental disorders caused by low BDNF levels.

3. Use of a compound having formula I according to claim 2 for the production of a drug for the treatment of lesions caused by CNS trauma.

4. Use of a compound having formula I according to any of claims 1 to 3, in which
R¹ is selected from the group consisting of octadec-9-enyl and geranylgeranyl group having formula:

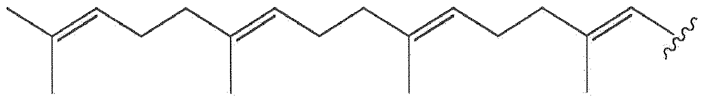

5. Use of a compound having formula I according to any of claims 1 to 4, in which R³ and R⁴ are both SO₃M, where M is an alkali metal selected from the group consisting of sodium and potassium.

6. Compound having formula I **characterised in that**
R¹ is an alkenyl residue C₅-C₂₅ containing one or more double carbon-carbon bonds,
R² is selected from the group consisting of methyl and fluorinated methyl, and
at least one of the substitutes R³ or R⁴ is an SO₃M group, in which M is selected from the group consisting of hydrogen, alkali metal, ammonium and quaternary amine, and the other substitute, if it is not an SO₃M group, is selected from the group consisting of hydrogen and acyl; provided that R² is methyl, and R³ and R⁴ are SO₃M groups.

7. Compound having formula I according to claim 6, in which R¹ is selected from the group consisting of octadec-9-enyl and geranylgeranyl having formula:

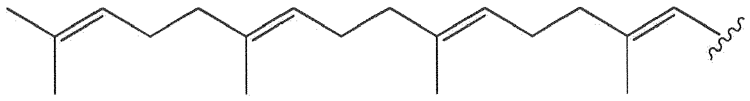

8. Compound having formula I according to either claim 6 or claim 7, in which R² is a trifluoromethyl group.

9. Method for producing the compound having formula I as described in any of claims 6 to 8, in which R³ and R⁴ are SO₃M and M is selected from the group consisting of hydrogen, alkali metal, ammonium and quaternary amine, **characterised in that** it comprises:
a) reaction of a compound having formula II with an alcohol having formula III in the presence of an acid to produce a compound having formula IV. 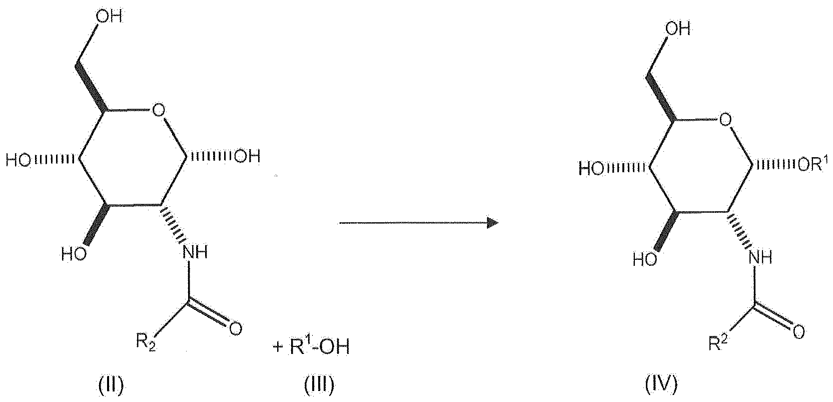 in which
R¹ is an alkenyl residue C₅-C₂₅ containing one or more double carbon-carbon bonds, and
R² is selected from the group consisting of methyl and fluorinated methyl, and
b) treating the compound VI with a sulphating agent to produce the compound having formula I in which R³ and R⁴ are SO₃M.

10. Method for producing the compound having formula I as described in any of claims 6 to 8, in which R³ is hydrogen, R⁴ is SO₃M and M is selected from the group consisting of hydrogen, alkali metal, ammonium and quaternary amine, **characterised in that** it comprises:
a') reaction of a compound having formula IV with 2,3-butaneodione and ethanol, in the presence of a protic acid to produce a compound having formula V, 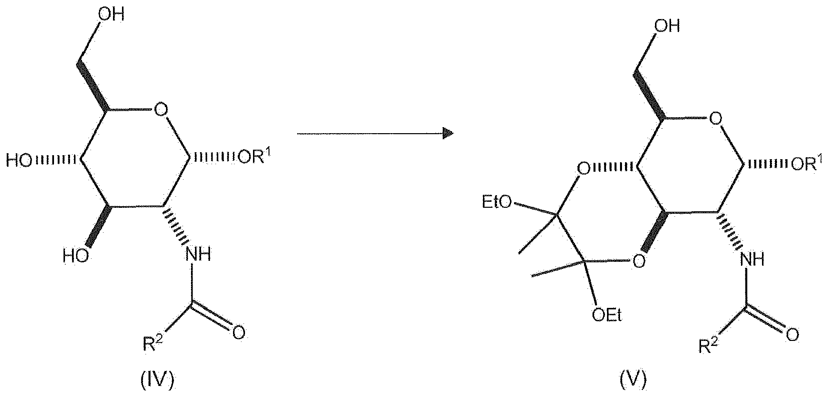 in which
R¹ is an alkenyl residue C₅-C₂₅ containing one or more double carbon-carbon bonds,
R² is selected from the group consisting of methyl and fluorinated methyl;
b') treating the compound V with a sulphating agent to produce the compound having formula VI; and 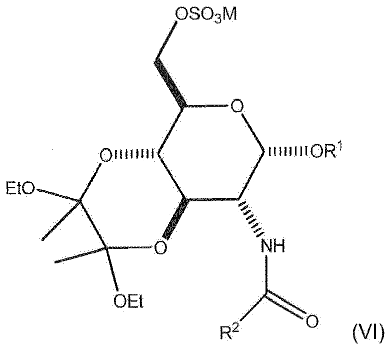
c') hydrolysing the compound VI to produce the compound having formula I in which R³ is hydrogen and R⁴ is SO₃M.

11. Method for producing the compound having formula I as described in any of claims 6 to 8, in which R³ is SO₃M, R⁴ is hydrogen and M is selected from the group consisting of hydrogen, alkali metal, ammonium and quaternary amine, **characterised in that** it comprises:
a") reaction of a compound having formula IV with 2,2-dimethoxypropane in the presence of a protic acid to produce a compound having formula VII; 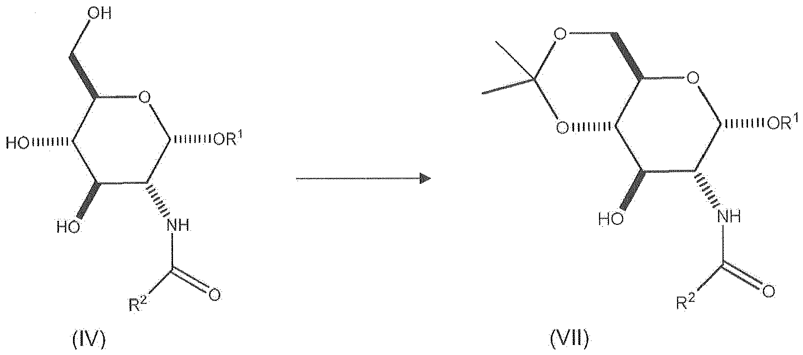 in which
R¹ is an alkenyl residue C₅-C₂₅ containing one or more double carbon-carbon bonds, and
R² is selected from the group consisting of methyl and fluorinated methyl;
b") treating the compound VII with a sulphating agent and hydrolysing the acetal group to produce the compound having formula I in which R³ is SO₃M and R⁴ is hydrogen.

12. Pharmaceutical composition which comprises a compound having formula I as described in any of claims 6 to 8.

13. Chemically marked derivative having formula VIII, in which M is selected from the group consisting of hydrogen, alkali metal, ammonium and quaternary amine.

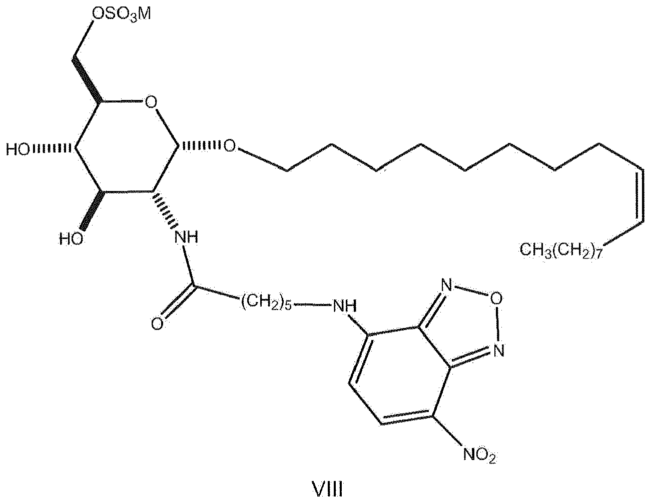

14. Method for producing the compound having formula VIII, **characterised in that** it comprises:
a) hydrolysing the compound having formula I in which R¹ is octadec-9-enyl, R² is trifluoromethyl, R³ is hydrogen and R⁴ is SO₃M to produce a compound having formula IX; 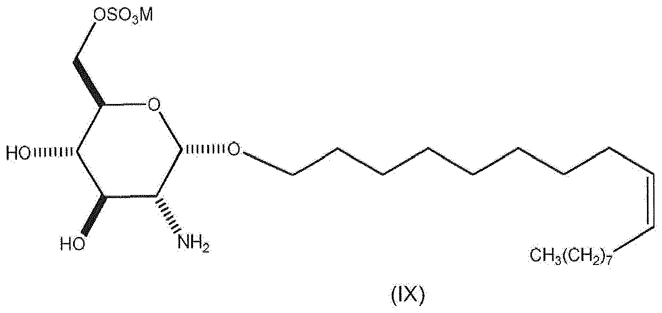
b) acylating the compound having formula IX with a compound having formula X to produce the compound having formula VIII.

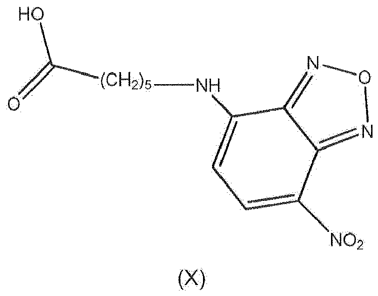

15. Analytical reagent which comprises the compound having formula I as described in any of claims 6 to 8, in which R² is fluorinated methyl, or a compound having formula VIII as described in claim 13.

16. Use of the compound having formula VIII as described in claim 13, to produce an analytical reagent for carrying out biological tests based on fluorescence detection.

17. Use of the compound having formula I as described in any of claims 6 to 8, in which R² is fluorinated methyl, to produce an analytical reagent for carrying out biological tests based on fluorine detection.
